# EUROPEAN PATENT SPECIFICATION

(11) **EP 1 288 664 B1**
(45) Date of publication and mention of the grant of the patent: **07.03.2007**
(21) Application number: 02256004.9
(22) Date of filing: 29.08.2002
(51) Int. Cl.: G01N 33/543, B01J 19/00

(54) **Methods for generating ligand arrays**
Verfahren zur Herstellung von Liganden-Arrays
Procédé de fabrication d'arrays de ligands

(30) Priority: 31.08.2001 US 944083
(43) Date of publication of application: 05.03.2003
(73) Proprietor: Agilent Technologies, Inc., Santa Clara, CA 95051 (US)
(72) Inventor: Lefkowitz, Steven M., Branford, CT 06405 (US); Kim, Namyong, North Andover, MA 01845 (US); Holcomb, Nelson R., San Jose, CA 95112 (US); Hargreaves, John S., Mountain View, CA 94040 (US); Dellinger, Geraldine F., Sunnyvale, CA 94087 (US); Dellinger, Douglas J., Sunnyvale, CA 94087 (US)
(74) Representative: Tollett, Ian

(56) References cited:
- US-B1- 6 258 454
- MASKOS U ET AL: "OLIGNUCLEOTIDE HYBRIDISATIONS ON GLASS SUPPORTS: A NOVEL LINKER FOROLIGONUCLEOTIDE SYNTHESIS AND HYBRIDISATION PROPERTIES OF OLIGNUCLEOTIDES SYNTHESISED IN SITU" NUCLEIC ACIDS RESEARCH, OXFORD UNIVERSITY PRESS, SURREY, GB, vol. 20, no. 7, 1992, pages 1679-1684, XP000651031 ISSN: 0305-1048

## Description

The field of this invention is ligand arrays, including protein and nucleic acid arrays. The invention relates to a method of covalently bonding a ligand to a substrate.

Arrays of binding agents (ligands), such as nucleic acids and polypeptides, have become an increasingly important tool in the biotechnology industry and related fields. These binding agent or ligand arrays, in which a plurality of binding agents are positioned on a solid support surface in the form of an array or pattern, find use in a variety of applications, including gene expression analysis, drug screening, nucleic acid sequencing, mutation analysis, and the like.

A feature of many arrays that have been developed is that each of the polymeric compounds of the array is stably attached to a discrete location on the array surface, such that its position remains constant and known throughout the use of the array. Stable attachment is achieved in a number of different ways, including covalent bonding of the polymer to the support surface and non-covalent interaction of the polymer with the surface.

Where the ligands of the arrays are polymeric, e.g., as is the case with nucleic acid and polypeptide arrays, there are two main ways of producing such arrays, i.e., via in situ synthesis in which the polymeric ligand is grown on the surface of the substrate in a step-wise fashion (known in the art as "growing from") and via deposition of the full ligand ("grafting to"), e.g., a presynthesized nucleic acid/polypeptide, cDNA fragment, etc., onto the surface of the array. In many situations where the desired polymeric ligands are long, the latter protocol of depositing full ligands on the substrate surface is desirable.

A number of different protocols have been developed in which full ligands are deposited onto the surface of an array, where such methods include those in which polylysine is adsorbed onto the surface of a glass support, those in which the surface of a glass support is modified via silylation to display various functional groups, and the like.

However, there is continued interest in the development of new protocols for producing arrays via deposition of full ligands onto the surface of the array. Of particular interest would be the development of protocols that provide for covalent attachment of full ligands, e.g., presynthesized nucleic acids, cDNAs and the like, following deposition of the full ligands on the support surface.

Patents and patent applications describing arrays of biopolymeric compounds and methods for their fabrication include: 5,242,974; 5,384,261; 5,405,783; 5,412,087; 5,424,186; 5,429,807; 5,436,327; 5,445,934; 5,472,672; 5,527,681; 5,529,756; 5,545,531; 5,554,501; 5,556,752; 5,561,071; 5,599,695; 5,624,711; 5,639,603; 5,658,734; WO 93/17126; WO 95/11995; WO 95/35505; EP 742 287; and EP 799 897. Also of interest are WO 97/14706, WO 98/30575 and WO 01/09385.

US 6,258,454 in the name of the present proprietor discloses a method of treating a solid support having hydrophilic moieties on its surface with a derivatizing composition containing a mixture of silanes in order to produce a low surface energy functionalized surface.

Methods of producing ligand arrays, e.g., polypeptide and nucleic acid arrays are provided. In the subject methods, a substrate having a surface displaying olefinic functional groups, e.g., olefin groups having a single site of unsaturation (α (alpha) olefins), are modified such that the olefinic functional groups are converted to ligand reactive functional groups chosen from aldehyde or activated carboxylate ester groups. The resultant substrate is then contacted with, typically, at least two different ligands, e.g., via deposition of each different ligand onto a different region of the surface, resulting in covalent attachment of the contacted ligand to the surface via reaction with the ligand reactive functional groups present on the substrate surface. Ligand arrays produced via the subject methods demonstrate a number of desirable properties, e.g., nucleic acid arrays produced by the subject methods provide high signal intensity with low background in nucleic acid hybridization assays, etc.

### DEFINITIONS

The term "polymer" means any compound that is made up of two or more monomeric units covalently bonded to each other, where the monomeric units may be the same or different, such that the polymer may be a homopolymer or a heteropolymer. Representative polymers include peptides, polysaccharides, nucleic acids and the like, where the polymers may be naturally occurring or synthetic.

The term "peptide" as used herein refers to any polymer compound produced by amide formation between a α-carboxyl group of one amino acid and an α-amino group of another group.

The term "oligopeptide" as used herein refers to peptides with fewer than about 10 to 20 residues, *i.e.* amino acid monomeric units.

The term "polypeptide" as used herein refers to peptides with more than 10 to 20 residues.

The term "protein" as used herein refers to polypeptides of specific sequence of more than about 50 residues.

The term "nucleic acid" as used herein means a polymer composed of nucleotides, e.g. deoxyribonucleotides or ribonucleotides, or compounds produced synthetically (e.g. PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "polynucleotide" as used herein refers to single or double stranded polymer composed of nucleotide monomers of generally greater than 100 nucleotides in length.

The term "functionalization" as used herein relates to modification of a solid substrate to provide a plurality of functional groups on the substrate surface. By a "functionalized surface" as used herein is meant a substrate surface that has been modified so that a plurality of functional groups are present thereon.

The terms "reactive site" or "reactive group" refer to moieties that can be used as the starting point in a synthetic organic process. This is contrasted to "inert" hydrophilic groups that could also be present on a substrate surface, e.g, hydrophilic sites associated with polyethylene glycol, a polyamide or the like.

The "surface energy" γ (measured in ergs/cm²) of a liquid or solid substance pertains to the free energy of a molecule on the surface of the substance, which is necessarily higher than the free energy of a molecule contained in the interior of the substance; surface molecules have an energy roughly 25% above that of interior molecules. The term "surface tension" refers to the tensile force tending to draw surface molecules together, and although measured in different units (as the rate of increase of surface energy with area, in dynes/cm), is numerically equivalent to the corresponding surface energy. By modifying a substrate surface to "reduce" surface energy is meant lowering the surface energy below that of the unmodified surface.

The term "monomer" as used herein refers to a chemical entity that can be covalently linked to one or more other such entities to form an polymer. Examples of "monomers" include nucleotides, amino acids, saccharides, peptoids, other reactive organic molecules and the like. In general, the monomers used in conjunction with the present invention have first and second sites (e.g., C-termini and N-termini(for proteins), or 5' and 3' sites(for oligomers, RNA's, cDNA's, and DNA's)) suitable for binding to other like monomers by means of standard chemical reactions (e.g., condensation, nucleophilic displacement of a leaving group, or the like), and a diverse element which distinguishes a particular monomer from a different monomer of the same type (e.g., an amino acid side chain, a nucleotide base, etc.). In the art synthesis of biomolecules of this type utilize an initial substrate-bound monomer that is generally used as a building-block in a multi-step synthesis procedure to form a complete ligand, such as in the synthesis of oligonucleotides, oligopeptides, and the like.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other polynucleotides which are C-glycosides of a purine or pyrimidine base, polypeptides (proteins), polysaccharides (starches, or polysugars), and other chemical entities that contain repeating units of like chemical structure. In the practice of the instant invention, oligomers will generally comprise about 2-50 monomers, preferably about 2-20, more preferably about 3-10 monomers.

The term "ligand" as used herein refers to a moiety that is capable of covalently or otherwise chemically binding a compound of interest. The arrays of solid-supported ligands produced by the subject methods can be used in screening or separation processes, or the like, to bind a component of interest in a sample. The term "ligand" in the context of the invention may or may not be an "oligomer" as defined above. However, the term "ligand" as used herein may also refer to a compound that is "presynthesized" or obtained commercially, and then attached to the substrate.

The term "sample" as used herein relates to a material or mixture of materials, typically, although not necessarily, in fluid form, containing one or more components of interest.

The terms "nucleoside" and "nucleotide" are intended to include those moieties which contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

As used herein, the term "amino acid" is intended to include not only the L-, D- and nonchiral forms of naturally occurring amino acids (alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine), but also modified amino acids, amino acid analogs, and other chemical compounds which can be incorporated in conventional oligopeptide synthesis, e.g., 4-nitrophenylalanine, isoglutamic acid, isoglutamine, ε-nicotinoyl-lysine, isonipecotic acid, tetrahydroisoquinoleic acid, α-aminoisobutyric acid, sarcosine, citrulline, cysteic acid, t-butylglycine, t-butylalanine, phenylglycine, cyclohexylalanine, β-alanine, 4-aminobutyric acid, and the like.

The terms "protection and "deprotection" as used herein relate, respectively, to the addition and removal of chemical protecting groups using conventional materials and techniques within the skill of the art and/or described in the pertinent literature; for example, reference may be had to Greene et al., Protective Groups in Organic Synthesis, 2nd Ed., New York: John Wiley & Sons, 1991. Protecting groups prevent the site to which they are attached from participating in the chemical reaction to be carried out.

The term "alkyl" as used herein refers to a branched or unbranched saturated hydrocarbon group of 1 to 24 carbon atoms, such as methyl, ethyl, *n*-propyl, isopropyl, *n*-butyl, isobutyl, *t*-butyl, octyl, decyl, tetradecyl, hexadecyl, eicosyl, tetracosyl and the like, as well as cycloalkyl groups such as cyclopentyl, cyclohexyl and the like. The term "lower alkyl" intends an alkyl group of 1 to 6 carbon atoms, preferably 1 to 4 carbon atoms.

The term "alkoxy" as used herein refers to a substituent -O-R wherein R is alkyl as defined above. The term "lower alkoxy" refers to such a group wherein R is lower alkyl.

The term "alkylene" as used herein refers to a difunctional saturated branched or unbranched hydrocarbon chain containing from 1 to 24 carbon atoms, and includes, for example, methylene (-CH2-), ethylene (-CH2-CH2-), propylene (-CH2-CH2-CH2-), 2-methylpropylene (-CH2-CH(CH3)-CH2-), hexylene (-(CH2)6-), and the like. "Lower alkylene" refers to an alkylene group of 1 to 6, more preferably 1 to 4, carbon atoms.

The terms "alkenyl" and "olfenic" as used herein refer to a branched or unbranched hydrocarbon group of 2 to 24 carbon atoms containing at least one carbon-carbon double bond, such as ethenyl, n-propenyl, isopropenyl, n-butenyl, isobutenyl, t-butenyl, octenyl, decenyl, tetradecenyl, hexadecenyl, eicosenyl, tetracosenyl and the like.

The terms "halogen" or "halo" are used in the conventional sense to refer to a chloro, bromo, fluoro or iodo substituent.

"Optional" or "optionally" means that the subsequently described circumstance may or may not occur, so that the description includes instances where the circumstance occurs and instances where it does not. For example, the phrase "optionally substituted" means that a non-hydrogen substituent may or may not be present, and, thus, the description includes structures wherein a non-hydrogen substituent is present and structures wherein a non-hydrogen substituent is not present.

Methods of producing ligand arrays, e.g., polypeptide and nucleic acid arrays are provided. In the subject methods, a substrate having a surface displaying olefinic functional groups, e.g., olefin groups having a single site of unsaturation, are modified such that the olefinic functional groups are converted to ligand reactive functional groups chosen from aldehyde or activated carboxylate ester group. The resultant substrate is then contacted with, typically, at least two different ligands, e.g., via deposition of each different ligand onto a different region of the surface, resulting in covalent attachment of the contacted ligand to the surface via reaction with the ligand reactive functional groups. Ligand arrays produced via the subject methods demonstrate a number of desirable properties, e.g., nucleic acid arrays produced by the subject methods provide high signal intensity with low background in nucleic acid hybridization assays, etc. In further describing the subject invention, the subject methods will be described first, followed by a review of the features of the arrays produced by the subject methods, as well as a description of representative uses for the subject arrays and kits the that include the subject arrays.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

### METHODS

As summarized above, the subject invention provides methods of producing ligand arrays. In the subject methods, the first step is to provide a substrate having a surface that displays olefinic functional groups. Next, the olefinic functional groups are converted to ligand reactive functional groups that, upon contact with a ligand, react to produce a covalent bond between the ligand and the substrate surface. Said ligand reactive functional groups are a aldehyde or an activated carboxylate ester group. Following this conversion step, the substrate surface is contacted with the ligands resulting in covalent linkage of the ligands to the surface so as to produce a ligand array. Each of these steps is now described in greater detail below.

### Providing a Substrate Having a Surface Displaying Olefinic Functional Groups

The first step in the subject methods is to provide a substrate having a surface that displays olefinic functional groups. Such a substrate may be provided using any convenient protocol. One way to provide such a substrate is to employ the following protocol.

In this protocol, the surface of a solid substrate is first contacted with a derivatizing composition that contains one or more types of silanes, where in many but not all embodiments the composition contains a mixture of silanes, under reaction conditions effective to couple the silanes to the substrate surface via reactive hydrophilic moieties present on the substrate surface. The reactive hydrophilic moieties on the substrate surface are typically hydroxyl groups, carboxyl groups, aldehyde, thiol groups, and/or substituted or unsubstituted amino groups, although, preferably, the reactive hydrophilic moieties are hydroxyl groups. The substrate may comprise any material that has a plurality of reactive hydrophilic sites on its surface, or that can be treated or coated so as to have a plurality of such sites on its surface. Suitable materials include, but are not limited to, supports that are typically used for solid phase chemical synthesis, e.g., cross-linked polymeric materials (e.g., divinylbenzene styrene-based polymers), agarose (e.g.SEPHAROSE^{™}), dextran (e.g., SEPHADEX^{™}), cellulosic polymers, polyacrylamides, silica, glass (particularly controlled pore glass, or "CPG") ceramics, and the like. The supports may be obtained commercially and used as is, or they may be treated or coated prior to functionalization. The substrate is typically flat with the contacted surface being planar (although these are not requirements).

The derivatizing composition contains at least one type of silane, where the silane includes an olefinic functional group, as described in greater detail below. In many embodiments, the derivatizing composition may include two types of silanes, a first silane that may be represented as R¹-Si(R^{L}R^{x}R^{y}) and a second silane having the formula R²-(L)ₙ-Si(R^{L}R^{x}R^{y}). In these formulae, the R^{L}, which may be the same or different, are leaving groups, the R^{x} and R^{y}, which may be the same or different, are either lower alkyl or leaving groups like R^{L}, R¹ is a chemically inert moiety that upon binding to the substrate surface lowers the surface energy thereof, n is 0 or 1, L is a linking group, and R² is a functional group enabling covalent binding of a molecular moiety or a group that may be modified to provide such a functional group. Reaction of the substrate surface with the derivatizing composition is carried out under reaction conditions effective to couple the silanes to the surface hydrophilic moieties and thereby provide -Si-R¹ groups and -Si-(L)ₙ-R² groups on the substrate surface.

More specifically, the R^{L} moieties, which are leaving groups, are such that they enable binding of the silanes to the surface. Typically, the leaving groups are hydrolyzable so as to form a silanol linkage to surface hydroxyl groups. Examples of suitable leaving groups include, but are not limited to, halogen atoms, particularly chloro, and alkoxy moieties, particularly lower alkoxy moieties. The R^{x} and R^{y} are either lower alkyl, e.g., methyl, ethyl, isopropyl, *n*-propyl, *t*-butyl, or the like, or leaving groups as just described with respect to R^{L}. Thus, each type of silane will generally contain a trichlorosilyl functionality, a tri(lower)alkoxysilyl functionality such as trimethoxysilyl, mixed functionalities such as diisopropylchlorosilyl, dimethylchlorosilyl, ethyldichlorosilyl, methylethylchlorosilyl or the like.

In these embodiments where a mixture of silanes make up the derivatizing composition, the first silane is a derivatizing agent that reduces surface energy as desired, while the second silane provides the olefinic functionality. Thus, with respect to the first silane, coupling to the substrate yields surface -Si-R¹ groups as explained above, wherein R¹ is a chemically inert moiety that upon binding to the substrate surface lowers surface energy. By "chemically inert"is meant that R¹ will not be cleaved or modified when the functionalized substrate is used for its intended purpose, e.g., in solid phase chemical synthesis, hybridization assays, or the like. Typically, R¹ is an alkyl group, generally although not necessarily containing in the range of 2 to 24 carbon atoms, preferably in the range of 10 to 18 carbon atoms. R¹ may also be benzyl, either unsubstituted or substituted with 1 to 5, typically 1 to 3, halogen, preferably fluoro, atoms.

The second silane, upon coupling, provides surface -Si-(L)ₙ-R² groups, where R² is the olefinic functionality. Of course, if the R^{x} and R^{y} are not leaving groups, the surface moieties provided will actually be -SiR^{x}R^{y}-(L)ₙ-R² groups, which applicants intend to encompass by the more generic representation -Si-(L)ₙ-R². R² in many embodiments includes a terminal -CH=CH₂ group, which can readily be converted to a ligand reactive group, e.g. a reactive hydroxyl group by boration and oxidation, as described in greater detail infra. L represents a linker and n is 0 or 1, such that a linker may or may not be present. If a linker is present, it will generally be a C₁-C₂₄ hydrocarbylene linking group. Normally, L is C₁-C₂₄ alkylene, preferably C₁₀-C₁₈ alkylene.

The density of R² olefinic functional groups on the substrate surface, following reaction with the derivatizing composition, is determined by the relative proportions of the first and second silanes in the derivatizing composition. That is, a higher proportion of the second silane in the derivatizing composition will provide a greater density of R² groups, while a higher proportion of the first silane will give rise to a lower density of R² groups. Optimally, the first silane represents in the range of approximately 0.5 wt.% to 50 wt.% of the derivatization composition, preferably in the range of approximately 1.0 wt.% to 10 wt.% of the composition, while the second silane correspondingly represents in the range of approximately 50 wt.% to 99.5 wt.% of the derivatization composition, preferably in the range of approximately 90 wt.% to 99 wt.% of the composition.

The resultant surface of the functionalized substrates contain both -Si-R¹ and Si-(L)ₙ-R² groups, present at a predetermined ratio, with the ratio determining both surface energy and density of functional groups. In other words, the functional surface of the substrate displays olefinic functional groups. See also U.S. Patent No. 6,258,454.

### Conversion of Olefenic Functional Groups to Ligand Reactive Functional Groups

The next step in the subject methods is to convert the olefinic functional groups on the surface of the subject to ligand reactive functional groups, chosen from a aldehyde group or an activated carboxylate ester group.

The particular conversion protocol employed will vary with respect to the nature of the desired ligand reactive functional group, and may or may not involve the production of one or more intermediate groups. Typically, the protocol employed is an oxidative protocol, where representative reactions that find use include, but are not limited to: ozonolysis; permanganate oxidation; hydroboration, OsO₄ oxidation/bisulfite reduction, hypobrimite oxidation; and the like. Representative protocols are provided in greater detail immediately below.

In one embodiment, the olefinic functional groups of the initial substrate surface are converted to aldehyde reactive functional groups. One protocol that may be employed is to covert the initial olefinic groups directly to aldehyde groups via ozonolysis. In these embodiments, the surface of the substrate is exposed to gaseous ozone or an ozone solution, followed by decomposition of the resultant olefine-ozone adduct (ozonide) with any of a variety of reagents, including, but not limited to: zinc+acetic acid, trimethyl phosphite, thiourea and dimethyl sulfide, which decomposition reagents may be gaseous or liquid. Such, processes are well known to those of skill in the art. See e.g., March, Advanced Organic Chemistry (1992) (4^{th} ed. John Wiley & Sons) pp 1177-1178. In yet other embodiments, the olefinic groups are first converted to intermediary hydroxyl groups that are then, in turn, converted to aldehyde groups. For example, the boration/oxidation reaction described in the experimental section, infra, can be employed to convert the surface olefinic functional groups to intermediary hydroxyl groups. These resultant intermediary surface hydroxyl groups can then be converted by controlled oxidation to aldehyde functionalities, e.g., via Moffat oxidations, where primary alcohols are specifically and efficiently converted to the corresponding aldehydes under mild conditions. See e.g., Pftizner and Moffatt, Comp. Org Syn. 7, 291 (1991), J. Amer. Chem. Soc. (1965) 87:5670-78. In yet another embodiment, the intermediary surface hydroxyl groups are converted to amine reactive benzaldehyde functionalities using benzaldehyde phosphoramidites. More specifically, the hydroxyl moiety can be reacted with a benzaldehyde phosphoramidite, followed by acidic deprotection of the benzaldehyde moiety and basic deprotection of the phosphate moiety. Such protocols are known in the art, see e.g., WO 01/09385 and its priority application serial no. 09/ 364,320. The olefinic moieties can also be converted to other functionalities using an analogous procedure with the appropriate phosphoramidite reagent.

In another embodiment, the olefinic moieties are converted to activated carboxylate esters. For example, permanganate oxidation, see e.g., Yu Tai et al., Bull. Inst. Chem. Academica Sinica (1988) 35: 23, is employed to convert the olefin to a carboxylic acid. The resultant carboxylic acid may then be activated using any convenient protocol, e.g., reaction with carbodiimide and N-hydroxysuccinimide, as is known in the art, so that the carboxylic acid is reactive to functional groups present on the ligand. See e.g., See e.g., Hermanson, (Bioconjugate Techniques (Academic Press, 1996) p. 139-140; and Stryer, Biochemistry(Third Ed. 1988), pg. 65.

While the particular results achieved may vary, the percentage of olefin functional groups that are converted is, in many embodiments, at least about 5%, usually at least about 10% and more usually at least about 20 number %, where the number % may be higher, e.g., 30, 40, 50, 60, 70, 80, 90, 95, 99.

### Ligand Attachment

The third step in the subject methods is ligand attachment. The ligands that are contacted with the substrate surface are typically polymeric binding agents. The polymeric binding agents may vary widely, where the only limitation is that the polymeric binding agents are made up of two more, usually a plurality of, monomeric units covalently attached in sequential order to one another such that the polymeric compound has a sequence of monomeric units. Typically, the polymeric binding agent includes at least 5 monomeric units, usually at least 10 monomeric units and more usually at least 15 monomeric units, where in many embodiments the number of monomeric units in the polymers may be as high as 5000 or higher, but generally will not exceed about 2000. In certain embodiments, the number of monomeric residues in the polymeric binding agent is at least about 50, usually at least about 100 and more usually at least about 150.

Polymeric binding agents of particular interest include biopolymeric molecules, such as polypeptides, nucleic acids, polysaccharides and the like, where polypeptides and nucleic acids, as well as synthetic mimetics thereof, are of particular interest in many embodiments.

In many embodiments, the polymeric binding agents are nucleic acids, including DNA, RNA, nucleic acids of one or more synthetic or non-naturally occurring nucleotides, and the like. The nucleic acids may be oligonucleotides, polynucleotides, including cDNAs, mRNAs, and the like. Where the polymeric compounds are nucleic acids, the nucleic acids will generally be at least about 5 nt, usually at least about 10 nt and more usually at least about 15 nt in length, where the nucleic acids may be as long as 5000 nt or longer, but generally will not exceed about 3000 nt in length and usually will not exceed about 2000 nt in length. In many embodiments, the nucleic acids are at least about 25 nt in length, usually at least about 50 nt in length and may be at least about 100 nt in length.

The polymers are characterized by having a functional moiety that reacts with the ligand reactive functional moiety present on the substrate surface to produce a covalent bond between the ligand and the substrate surface. The ligand may naturally include the desired reactive functionality, or may be modified to include the desired reaction functionality. Representative reactive functionalities of interest include, but are not limited to: amine groups, hydroxyl groups, sulfhydryl, phosphoramidite, anhydrides, and the like.

The polymers employed in the subject methods may be prepared using any convenient methodology. The particular means of preparing the polymer to include the requisite reactive group where it is not initially present will depend on the nature of the polymer and the nature of the reactive group that is to be incorporated into the polymer.

As mentioned above, in practicing the subject methods, typically at least two distinct polymers are contacted with the substrate surface that bears the reactive ligand functionalities. By distinct is meant that the two polymers differ from each other in terms of sequence of monomeric units. The number of different polymers that are contacted with the substrate surface may vary depending on the desired nature of the array to be produced, i.e. the desired density of polymeric structures. Generally, the number of distinct polymers that are contacted with the surface of the array will be at least about 5, usually at least about 10 and more usually at least about 100, where the number may be as high as 1,000,000 or higher, but in many embodiments will not exceed about 500,000 and in certain embodiments will not exceed about 100,000.

The polymers are generally contacted with the surface in an aqueous solvent, such that aqueous conditions are established at the surface location to which the polymer is to be covalently attached. The temperature during contact typically ranges from about 10 to 60 and usually from about 20 to 40 °C. Following initial contact, the aqueous solution of polymer is typically maintained for a period of time sufficient for the desired amount of reaction to occur, where the period of time is typically at least about 20 sec, usually at least about 1 min and more usually at least about 10 min, where the period of time may be as great as 20 min or greater.

Each polymer is typically contacted with the substrate surface as part of an aqueous composition, i.e. an aqueous composition of the polymer in an aqueous solvent is contacted with the surface of the array. The aqueous solvent may be either water alone or water in combination with a co-solvent, e.g. an organic solvent, and the like. The aqueous composition may also contain one or more additional agents, including: acetic acid, monochloro acetic acid, dichloro acetic acid, trichloro acetic acid, acetonitrile, catalysts, e.g. lanthanide (III) trifluoromethylsulfate, lithium chloride, buffering agents, e.g. sodium phosphate, salts, metal cations, surfactants, enzymes, etc.

The aqueous polymer composition may be contacted with the surface using any convenient protocol. Generally, the aqueous polymer composition is contacted with the surface by depositing the aqueous polymer composition on the surface of the substrate. The aqueous volume may be deposited manually, e.g. via pipette, or through the use of an automated machine or device. A number of devices and protocols have been developed for depositing aqueous solutions onto precise locations of a support surface and may be employed in the present methods. Such devices include "ink-jet" printing devices, mechanical deposition or pipetting devices and the like. See e.g. U.S. Patent Nos. 4,877,745; 5,338,688; 5,474,796; 5,449,754; 5,658,802; 5,700,637; and 5,807,552; the disclosures of which are herein incorporated by reference. Robotic devices for precisely depositing aqueous volumes onto discrete locations of a support surface, i.e. arrayers, are also commercially available from a number of vendors, including: Genetic Microsystems; Cartesian Technologies; Beecher Instruments; Genomic Solutions; and BioRobotics.

The amount of fluid that is deposited may vary. For example, volumes ranging from about 1nl to 1 pl, usually from about 60 to 100 nl may be deposited onto the substrate surface. Following contact and incubation for a period of time and under conditions sufficient for the desired reaction to occur, as described above, the surface of the resultant array may be further processed as desired in order to prepare the array for use, as described below. As such, the array surface may be washed to remove unbound reagent, e.g. unreacted polymer, and the like. Any convenient wash solution and protocol may be employed, e.g. flowing an aqueous wash solution, e.g. water, methanol, acetonitrile, and the like, across the surface of the array, etc. The surface may also be dried and stored for subsequent use, etc.

The above protocol produces ligand arrays that can be employed in a variety of different applications, as described in greater detail infra.

### UTILITY

The arrays produced by the subject methods find use in a variety applications, where such applications are generally analyte detection applications in which the presence of a particular analyte in a given sample is detected at least qualitatively, if not quantitatively. Protocols for carrying out such assays are well known to those of skill in the art and need not be described in great detail here. Generally, the sample suspected of comprising the analyte of interest is contacted with an array produced according to the subject methods under conditions sufficient for the analyte to bind to its respective binding pair member that is present on the array. Thus, if the analyte of interest is present in the sample, it binds to the array at the site of its complementary binding member and a complex is formed on the array surface. The presence of this binding complex on the array surface is then detected, e.g. through use of a signal production system, e.g. an isotopic or fluorescent label present on the analyte, etc. The presence of the analyte in the sample is then deduced from the detection of binding complexes on the substrate surface.

Specific analyte detection applications of interest include hybridization assays in which the nucleic acid arrays of the subject invention are employed. In these assays, a sample of target nucleic acids is first prepared, where preparation may include labeling of the target nucleic acids with a label, e.g. a member of signal producing system. Following sample preparation, the sample is contacted with the array under hybridization conditions, whereby complexes are formed between target nucleic acids that are complementary to probe sequences attached to the array surface. The presence of hybridized complexes is then detected. Specific hybridization assays of interest which may be practiced using the arrays prepared by the method of the invention include: gene discovery assays, differential gene expression analysis assays; nucleic acid sequencing assays, and the like. Patents and patent applications describing methods of using arrays in various applications include: 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; WO 95/21265; WO 96/31622; WO 97/10365; WO 97/27317; EP 373 203; and EP 785 280.

In gene expression analysis with microarrays, an array of "probe" nucleic acids is contacted with a nucleic acid sample of interest. Contact is carried out under hybridization conditions and unbound nucleic acid is then removed. The resultant pattern of hybridized nucleic acid provides information regarding the genetic profile of the sample tested. Gene expression analysis finds use in a variety of applications, including: the identification of novel expression of genes, the correlation of gene expression to a particular phenotype, screening for disease predisposition, identifying the effect of a particular agent on cellular gene expression, such as in toxicity testing; among other applications.

In certain embodiments, the subject methods include a step of transmitting data from at least one of the detecting and deriving steps, as described above, to a remote location. The data may be raw data (such as fluorescence intensity readings for each feature in one or more color channels) or may be processed data such as obtained by rejecting a reading for a feature which is below a predetermined threshold and/or forming conclusions based on the pattern read from the array (such as whether or not a particular target sequence may have been present in the sample). By "remote location" is meant a location other than the location at which the array is present and hybridization occur. For example, a remote location could be another location (e.g. office, lab, etc.) in the same city, another location in a different city, another location in a different state, another location in a different country, etc. The data may be transmitted or otherwise forwarded to the remote location for further evaluation and/or use. Any convenient telecommunications means may be employed for transmitting the data, e.g., facsimile, modem, internet, etc.

The following examples are offered by way of illustration and not by way of limitation.

### EXPERIMENTAL

### Example 1. Preparation of Functionalized Surfaces:

This example describes functionalization of a glass substrate with a derivatizing composition comprising 97.5 wt.% *n*-decyltrichlorosilane ("NTS") as a first silane and 2.5 wt.% undecenyltrichlorosilane ("UTS") as a second silane, followed by boration and oxidation to convert the terminal olefinic moiety of the surface-bound UTS to a hydroxyl group.

### (a) Silylation:

Under moisture-free conditions, 14 ml NTS and 0.4 ml UTS were added to 800 ml of anhydrous toluene, and swirled to mix. Cleaned glass substrates were placed into a ca. 1 liter reactor equipped for inert gas purging, heating and stirring, and purging was conducted for 30 minutes. Moisture-free conditions were maintained, and the NTS/UTS solution was added to the reactor. The solution was heated to 100°C for 4 hours, while stirring and continuing to maintain moisture-free conditions. The silane solution was removed from the reactor and replaced with anhydrous toluene. This step was repeated twice.

The substrates were then removed from the reactor and rinsed rigorously with an appropriate solvent. The bulk solvent was removed from the substrates by blowing with clean inert gas. The substrates were placed in a vacuum oven preheated to 150°C and heated under vacuum for 1 hour. The substrates were removed and allowed to cool to ambient temperature.

### (b) Boration and Oxidation:

The silylated substrates prepared in part (a) were placed in a ca. 1 liter reactor equipped for inert gas purging and stirring, and purging was conducted for 30 minutes. Under moisture-free conditions, 800 ml of 1.0 M borane-tetrahydrofuran complex was transferred to the reactor. The substrates were incubated while stirring, for two hours. Then, while maintaining moisture-free conditions, the boration solution was removed and replaced with 800 ml anhydrous tetrahydrofuran. The substrates were removed and rinsed rigorously with an appropriate solvent. Bulk solvent was removed by blowing with clean inert gas.

To a 1 liter vessel equipped for stirring, 800 ml of 0.1 N NaOH in 30% hydrogen peroxide (aqueous) was added. The oxidized substrates were immersed therein, and incubated, with stirring, for 10 minutes. The substrates were removed and rinsed rigorously with an appropriate solvent, then dried by blowing with clean inert gas.

The processes of steps (a) and (b) were repeated using different mole ratios of NTS and UTS, 100% UTS, and a mixture of glycidoxypropyl trimethoxysilane and hexaethylene glycol (GOPS-HEG). This hydroxyl silane-linker was prepared following the procedure of Maskos et al. (Maskos et al. (1992) *Nucleic Acids Res.* 20:1679) who demonstrated it to be useful for both oligonucleotide synthesis and hybridization.

### Example 2. Preparation of Arrays Via Deposition

Mixed undecenyl/decyl silane (UDS) films were prepared on glass slides, and the olefin moiety of the undecenyl silane was converted to a hydroxyl group by the hydroboration and oxidation protocol described above. The composition of the undecenyl silane in a mixed film varied from 2% to 100%, where higher undecenyl content needed longer exposure of a UDS film to oxidation. The hydroxyl groups of the resultant UDS substrates were further oxidized to adehyde groups following the procedure disclosed in Moffatt et al., supra, with modification. In the process, the substrates were immersed in 60 mmol dicyclohyexylcarbodiimide and 2 mmol anhydrous phosphoric acid in DMSO overnight under Ar atmosphere. After the exposure, the substrates were washed with DMF and EtOH subsequently and dried under nitrogen.

The resultant modified UDS slides were used as substrates for presynthesized-oligonucleotides deposition microarrays, where amine terminated oligonucleotides were spotted onto the slides.

The microarray surfaces were evaluated using 10 probes total including amine and non-amine terminated yeast and ref seq 25mer and 60mers using typical spotting buffer. After spotting the oligomers on the substrate they were passivated using techniques which generate nonreactive surface functional groups. Both UDS and Telechem slides were post-processed using the aldehyde passivation protocol familiar to those versed in the art (reduction with NaBH4).

Hybridization of probes on the surface were carried out according to the following general protocol outline:
Targets: 5 µg/mL Cy3 K562, 5 µg/mL Cy5 Hela (both of which were labeled using the linear amplification kit), and 30pM YER targets (direct labeled).
Targets were fragmented using Zn fragmentation buffer at 60 °C for one half hour. Hybridization was carried out in large volume (200 µL) chambers using typical pH 6.4 hybridization buffer. Duration of hybridization was 17 hours at 60°C. Slides were washed post hybridization using *in situ* SOP.

The resultant arrays on modified UDS slides exhibited high signal intensity and low background compared to those of Telechem Superaldehyde substrates. The UDS surface was oxidized to aldehyde functional groups using one of several oxidation methods, Moffat oxidation, ozonolysis, or permanganate. Assays were performed using above complex human targets (HELA and K562) along with a single yeast target. Hybridizations were carried out under typical conditions: 60°C, 17hr., in hybridization oven. Results for UDS showed a consistent 4-5 fold increase in signal intensities over identically hybridized TeleChem Superaldehyde surfaces. For example, DNA arrays on aldehyde terminated 100% UDS slides showed three times higher background-subtracted signals than Telechem arrays under identical experimental conditions.

## Claims

1. A method of covalently bonding a ligand to a substrate, said method comprising:
(a) providing a substrate having a surface displaying olefin functional groups;
(b) converting said olefin functional groups to ligand reactive functional groups that covalently bond to said ligand upon contact with said ligand; and
(c) contacting said surface with said ligand to covalently bond said ligand to said substrate,
**characterised in that** said ligand reactive functional group is an aldehyde or an activated carboxylate ester

2. A method as claimed in claim 1, wherein said method is a method of producing an array of at least two different ligands covalently bonded to a surface of a substrate, and said step (c) comprises contacting said surface with said at least two different ligands.

3. A method as claimed in claim 1 or 2, wherein said olefin functional groups consist of a single site of unsaturation.

4. A method as claimed in any preceding claim, wherein said ligands are polymers.

5. A method as claimed in claim 4, wherein said polymers are nucleic acids or peptides.

6. A method as claimed in any preceding claim, wherein said contacting step (c) comprises depositing each of said at least two different polymer ligands in a different region of said surface.

7. A method as claimed in any preceding claim, wherein said aldehyde is a benzaldehyde.

## Patentansprüche

1. Ein Verfahren zum kovalenten Binden eines Liganden an ein Substrat, wobei das Verfahren folgende Schritte umfasst:
(a) Bereitstellen eines Substrats, das eine Oberfläche aufweist, auf der olefinfunktionelle Gruppen vorliegen;
(b) Umwandeln der olefinfunktionellen Gruppen in ligandenreaktive funktionelle Gruppen, die sich auf einen Kontakt mit dem Liganden hin kovalent an den Liganden binden; und
(c) Inberührungbringen der Oberfläche mit dem Liganden, um den Liganden kovalent an das Substrat zu binden,
**dadurch gekennzeichnet, dass** die ligandenreaktive funktionelle Gruppe ein Aldehyd oder ein aktivierter Carboxylatester ist.

2. Ein Verfahren gemäß Anspruch 1, wobei das Verfahren ein Verfahren zum Erzeugen eines Arrays aus zumindest zwei unterschiedlichen Liganden ist, die kovalent an eine Oberfläche eines Substrats gebunden sind, und wobei der Schritt (c) ein Inberührungbringen der Oberfläche mit den zumindest zwei unterschiedlichen Liganden umfasst.

3. Ein Verfahren gemäß Anspruch 1 oder 2, bei dem die olefinfunktionellen Gruppen aus einer einzigen Stelle einer Ungesättigtheit bestehen.

4. Ein Verfahren gemäß einem der vorhergehenden Ansprüche, bei dem die Liganden Polymere sind.

5. Ein Verfahren gemäß Anspruch 4, bei dem die Polymere Nucleinsäuren oder Peptide sind.

6. Ein Verfahren gemäß einem der vorhergehenden Ansprüche, bei dem der Schritt (c) des Inberührungbringens ein Aufbringen jedes der zumindest zwei unterschiedlichen Polymerliganden in einer unterschiedlichen Region der Oberfläche umfasst.

7. Ein Verfahren gemäß einem der vorhergehenden Ansprüche, bei dem das Aldehyd ein Benzaldehyd ist.

## Revendications

1. Méthode permettant de lier de manière covalente un ligand à un substrat, ladite méthode comprenant :
(a) la fourniture d'un substrat ayant une surface présentant des groupes fonctionnels oléfiniques ;
(b) la conversion desdits groupes fonctionnels oléfiniques en groupes fonctionnels réactifs vis-à-vis du ligand qui se lient de manière covalente audit ligand lors d'un contact avec ledit ligand ; et
(c) la mise en contact de ladite surface avec ledit ligand pour lier de manière covalente ledit ligand audit substrat,
**caractérisée en ce que** ledit groupe fonctionnel réactif vis-à-vis du ligand est un aldéhyde ou un ester carboxylate activé.

2. Méthode selon la revendication 1, dans laquelle ladite méthode est une méthode permettant de produire un ensemble d'au moins deux ligands différents liés de manière covalente à une surface d'un substrat, et ladite étape (c) comprend la mise en contact de ladite surface avec lesdits au moins deux ligands différents.

3. Méthode selon la revendication 1 ou la revendication 2, dans laquelle lesdits groupes fonctionnels oléfiniques consistent en un seul site d'insaturation.

4. Méthode selon l'une quelconque des revendications précédentes, dans laquelle lesdits ligands sont des polymères.

5. Méthode selon la revendication 4, dans laquelle lesdits polymères sont des acides nucléiques ou des peptides.

6. Méthode selon l'une quelconque des revendications précédentes, dans laquelle ladite étape de mise en contact (c) comprend le dépôt de chacun desdits au moins deux ligands polymères différents dans une région différente de ladite surface.

7. Méthode selon l'une quelconque des revendications précédentes, dans laquelle ledit aldéhyde est un benzaldéhyde.
